(19) Europäisches Patentamt
European Patent Office
Office européen des brevets

(11) EP 3 532 012 B1

(12) **EUROPEAN PATENT SPECIFICATION**

(45) Date of publication and mention of the grant of the patent:
**16.03.2022 Bulletin 2022/11**

(21) Application number: **18734376.9**

(22) Date of filing: **05.06.2018**

(51) International Patent Classification (IPC):
***A61K 8/25*** $^{(2006.01)}$ ***A61K 8/27*** $^{(2006.01)}$
***A61K 8/34*** $^{(2006.01)}$ ***A61Q 5/00*** $^{(2006.01)}$
***A61Q 5/02*** $^{(2006.01)}$

(52) Cooperative Patent Classification (CPC):
**A61K 8/345; A61K 8/25; A61K 8/27; A61Q 5/008; A61Q 5/02**

(86) International application number:
**PCT/US2018/035996**

(87) International publication number:
**WO 2018/226651 (13.12.2018 Gazette 2018/50)**

(54) **HAIR CARE COMPOSITIONS COMPRISING MATERIALS THAT MODIFY SEBUM**

HAARPFLEGEZUSAMMENSETZUNGEN MIT MATERIALIEN, DIE SEBUM MODIFIZIEREN

COMPOSITIONS DE SOINS CAPILLAIRES COMPRENANT DES MATIÈRES QUI MODIFIENT LE SÉBUM

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.06.2017 US 201762515134 P**

(43) Date of publication of application:
**04.09.2019 Bulletin 2019/36**

(73) Proprietor: **The Procter & Gamble Company**
**Cincinnati, OH 45202 (US)**

(72) Inventors:
- **PUNYANI, Supriya**
  **Singapore 138547 (SG)**
- **PAUL, Monalisha**
  **Singapore 138547 (SG)**
- **JONES, Stevan, David**
  **Cincinnati, Ohio 45202 (US)**
- **PAGE, Steven, Hardy**
  **Cincinnati, Ohio 45202 (US)**
- **ISAACS, Sandra, Nichole**
  **Cincinnati, Ohio 45202 (US)**
- **CONSTANTINIDES, Ioannis, Constantine**
  **Cincinnati, Ohio 45202 (US)**
- **PELILLO, Enrico**
  **Singapore 138547 (SG)**

(74) Representative: **P&G Patent Germany**
**Procter & Gamble Service GmbH**
**Sulzbacher Straße 40**
**65824 Schwalbach am Taunus (DE)**

(56) References cited:
**EP-A1- 3 061 501**
**WO-A1-2011/047421**
**WO-A1-2017/027603**
**US-A1- 2015 118 172**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### FIELD OF THE INVENTION

The present disclosure relates to hair care compositions comprising 1,2-diols and hydrophobic particles that provide durable clean feel on hair and scalp and easy sebum removal upon subsequent shampoo cleansing.

### BACKGROUND OF THE INVENTION

Clean Scalp and Hair is described by consumers as having no sticky or greasy feel, no clumped fibers, no odor, and no hair weigh-down. Generally, consumers perceive unclean scalp and hair when sebum in liquid state builds up on their scalp and their hair during the end of the day. The liquid sebum on hair and scalp is often associated with unclean, greasy, oily and dirty look, feel and smell. Sebum is continuously secreted out of the sebaceous glands on scalp in liquid form. Due to dynamic environment (exposure to UV and microflora), sebum is unstable and its composition rapidly changes. As a result, it typically exists in more than one phase on the scalp and it gets transferred to hair during the day. Cleansing with surfactant-containing shampoo, removed approximately 60-90% of the accumulated sebum, depending on the surfactant concentration. Most of the shampoos remove on average 80% of the sebum. However, as sebum secretion is a continuous process, sebum re-appears on the scalp with significant amounts accumulating within 5-6 hours after shampooing. As mentioned above, sebum gets progressively transferred to the hair fibers, which leads to unclean consumer perception within 5-6 hours from the previous wash. Most consumers use surfactant-containing shampoos to clean their hair, whereas there is a minority of consumers who use oil-based products to clean their hair. It is perceived that many shampoos with high surfactant content strip the hair fiber surface from natural lubricants, leading to dry and squeaky hair feel.

WO 2011/047421 A1 refers to an antifungal composition for topical treatment of dermatitis associated with fungal infections comprising a polymer of 2-propenal and a dermatologically acceptable carrier.

US 2015/118172 A1 is related to the use of at least one dicyclohexylmethanol derivative of the formula (I) as antimicrobial active compound or as anti-acne, anti dandruff, deodorant or antiperspirant active compound.

EP 306150 1A1 sets out a cosmetic or dermatological composition comprising a combination of vegetal extracts titrated in salicin and 1,2-alkanediols, preferably 1, 2-decanediol and willow bark *(Salix alba)* extract, useful for the treatment of acne.

WO 2017/027603 A1 refers to methods and compositions useful for a topical composition comprising encapsulated resveratrol, oligopeptide-1, niacinamide, Opuntia ficus-indica extract, Primus mume extract, algae extract, malachite extract, adenosine, and/or Opuntia tuna fruit extract.

Thus, there is an undesired trade-off in shampooing and a need exists for hair care products that can effectively delay the appearance and feel of unclean scalp and hair as well as can remove sebum without the above-mentioned negative trade-offs.

Without wishing to be bounded by theory, the presence of the combination of these materials in hair care compositions provide for the benefits by modifying the sebum physical properties such as melting characteristic and/or absorbing sebum. As a result, the transfer of sebum from the scalp to hair fibers is reduced, making hair appearance and feel to be less unclean. In addition, treatment the hair and scalp with hair care compositions comprising the combination of the above-mentioned materials contributes to more effective sebum removal upon subsequent cleansing with shampoo.

### SUMMARY OF THE INVENTION

A pre-wash composition for clean benefit is provided and comprises:

a) From 0.1 wt.% to 12 wt.% of a 1,2-diol having a carbon chain with a length of more than 8 carbons;
b) From 0.1 wt.% to 10 wt.% of a solid particle, the solid particle is selected from the group consisting of silica silylate (hydrophobic silica), zinc carbonate and mixtures thereof, wherein the total amount of solid particle ranges from 1 wt.% to 10 wt.%, wherein

(1) the interfacial tension between the solid particle and sebum is from 5 to 18 dyn/cm as measured using the Contact Angle Method as disclosed herein;
(2) the sebum exhibits spreading coefficient on the solid, which is greater than 22 dyn/cm as measured using the Contact Angle Method as disclosed herein; and
(3) the work of adhesion of the sebum to the solid particle, which is greater than 75 dyn/cm as measured using the Contact Angle Method as disclosed herein.

c) an aqueous carrier; and

d) from 0.1 wt.% to 5 wt.% emulsifier selected from the group consisting of anionic, non-ionic, cationic and amphoteric

The product compositions of the present disclosure provide durable clean feel on hair and scalp and easy sebum removal upon subsequent shampoo cleansing.

BRIEF DESCRIPTION OF THE DRAWINGS

It is to be understood that both the foregoing general description and the following detailed description describe various non-limiting examples and are intended to provide an overview or framework for understanding the nature and character of the claimed subject matter. The accompanying drawings are included to provide a further understanding of various non-limiting examples, and are incorporated into and constitute a part of this specification. The drawings illustrate various non-limiting examples described herein, and together with the description serve to explain the principles and operations of the claimed subject matter.

FIG. 1 is a non-limiting example depicting the measuring of angle of contact.

DETAILED DESCRIPTION OF THE INVENTION

While the specification concludes with claims which particularly point out and distinctly claim the invention, it is believed the present invention will be better understood from the following description.

The present invention can comprise, consist of, or consist essentially of the essential elements and limitations of the invention described herein, as well any of the additional or optional ingredients, components, or limitations described herein.

All percentages and ratios used herein are by weight of the total composition, unless otherwise designated. All measurements are understood to be made at ambient conditions, where "ambient conditions" means conditions at 25 °C, under one atmosphere of pressure, and at 50% relative humidity (RH), unless otherwise designated. All numeric ranges are inclusive of narrower ranges; delineated upper and lower range limits are combinable to create further ranges not explicitly delineated.

The compositions of the present disclosure can comprise, consist essentially of, or consist of, the essential components as well as optional ingredients described herein. As used herein, "consisting essentially of" means that the composition or component may include additional ingredients, but only if the additional ingredients do not materially alter the basic and novel characteristics of the claimed compositions or methods.

"Apply" or "application" as used in reference to a composition, means to apply or spread the compositions of the present disclosure onto keratinous tissue such as the hair.

"Dermatologically acceptable" means that the compositions or components described are suitable for use in contact with human skin tissue without undue toxicity, incompatibility, instability, allergic response, and the like.

"Safe and effective amount" means an amount of a compound or composition sufficient to significantly induce a positive benefit.

"Leave-on," in reference to compositions, means compositions intended to be applied to and allowed to remain on the keratinous tissue. These leave-on compositions are to be distinguished from compositions, which are applied to the hair and subsequently (in a few minutes or less) removed either by washing, rinsing, wiping, or the like. Leave-on compositions exclude rinse-off applications such as shampoos, rinse-off conditioners, facial cleansers, hand cleansers, body wash, or body cleansers. The leave-on compositions may be substantially free of cleansing or detersive surfactants. For example, "leave-on compositions" may be left on the keratinous tissue for at least 15 minutes. For example, leave-on compositions may comprise less than 1% detersive surfactants, less than 0.5% detersive surfactants, or 0% detersive surfactants. The compositions may, however, contain emulsifying, dispersing or other processing surfactants that are not intended to provide any significant cleansing benefits when applied topically to the hair.

"Soluble" means at least 0.1 g of solute dissolves in 100 ml of solvent, at 25 °C and 1 atm of pressure.

All percentages are by weight of the total composition, unless stated otherwise. All ratios are weight ratios, unless specifically stated otherwise. All ranges are inclusive and combinable. The number of significant digits conveys neither a limitation on the indicated amounts nor on the accuracy of the measurements. The term "molecular weight" or "M.Wt." as used herein refers to the weight average molecular weight unless otherwise stated. The weight average molecular weight may be measured by gel permeation chromatography. "QS" means sufficient quantity for 100%.

The term "substantially free from" or "substantially free of" as used herein means less than 1%, or less than 0.8%, or less than 0.5%, or less than 0.3%, or 0%, by total weight of the composition.

"Hair," as used herein, means mammalian hair including scalp hair, facial hair and body hair, particularly on hair on the human head and scalp.

"Solid Particles", as used herein, means particle and/or powder blends that may be free flowing compositions

or suspensions of synthetic porous agglomerates comprising of organic and/or inorganic compounds.

"Cosmetically acceptable," as used herein, means that the compositions, formulations or components described are suitable for use in contact with human keratinous tissue without undue toxicity, incompatibility, instability, allergic response, and the like. All compositions described herein which have the purpose of being directly applied to keratinous tissue are limited to those being cosmetically acceptable.

"Derivatives," as used herein, includes but is not limited to, amide, ether, ester, amino, carboxyl, acetyl, acid, salt and/or alcohol derivatives of a given compound.

"Polymer," as used herein, means a chemical formed from the polymerisation of two or more monomers. The term "polymer" as used herein shall include all materials made by the polymerisation of monomers as well as natural polymers. Polymers made from only one type of monomer are called homopolymers. Polymers made from two or more different types of monomers are called copolymers. The distribution of the different monomers can be calculated statistically or block-wise - both possibilities are suitable for the present invention. Except if stated otherwise, the term "polymer" used herein includes any type of polymer including homopolymers and copolymers.

"Pre-Wash Aqueous Composition"

"Pre-Wash", in reference to compositions of the current invention means compositions intended to be used for applying the product before shampooing the hair. The pre-wash step may be followed by rinsing, wiping, of the like before shampooing. The pre-wash compositions may be substantially free of cleansing or detersive surfactants. For example, "Pre-wash compositions" may be left on the keratinous tissue for at least 2 minutes. For example, Pre-wash compositions may comprise less than 1% detersive surfactants, less than 0.5% detersive surfactants, or 0% detersive surfactants. The compositions may, however, contain emulsifying, dispersing or other processing surfactants that are not intended to provide any significant cleansing benefits when applied topically to the hair.

The hair care compositions of the present disclosure can be pre-wash composition, rinse-off conditioner, or leave-on treatment. They provide longevity of hair clean feel and appearance. They also provide excellent cleaning performance upon subsequent cleansing with shampoo without significantly negatively affecting hair feel. The benefit is achieved by the composition of sebum modifiers included in the hair care compositions. The combination of the sebum modifier thereof can modify the sebum physical properties such as melting characteristic and/or absorbing sebum so that sebum can be prevented from transferring from the scalp to hair fibers during the day. The above-mentioned benefit related to sebum removal is observed by measuring sebum removal from hair that is treated with hair care compositions that contain the inventive combination of materials and subsequently washed with shampoo compared to control hair that is not treated with the simple compositions but washed with the same shampoo.

SEBUM MODIFIER

The following classes of sebum modifiers or combinations thereof can modify the sebum physical properties such as melting characteristic and/or absorbing sebum.

a) 1,2 diols contain having a carbon chain with a length of more than 8 carbons; e.g. 1,2-decanediol, 1,2-dodecanediol, 1,2-octanediol for 1-2-diols.

b) hydrophobic solid particle or hydrophobically modified solid particle such as silica silylate, zinc carbonate, hydrophobic clay , zinc oxide, polyethylene powders, polypropylene powders, polystyrene powders, calcium silicate, polyethylene, nylon, boron nitride, mica, clays such as bentonite, montmorillonite and kaolin, zeolite, cyclodextrins, fumed silica, synthetic clays such as polymer powders including natural, synthetic, and semisynthetic cellulose, fluorocarbon resins, polypropylene, modified starches of cellulose acetate, particulate cross- linked hydrophobic acrylate or methacrylate copolymers and mixtures thereof. Starches hydrophobically modified to have a high capacity for loading oils. Such starches can be modified with alkyl or alkenyl substituted dicarboxylic acids. Such materials may contain counter-ions, for example metals such as aluminum. A preferred such material is Natrasorb HFB available from National Starch and Chemical Company, U.S. A, which contains aluminum starch octenyl succinate. Other suitable materials from National Starch and Chemical include Natrasorb Bath, Dry-Flow PC, Dry-How XT, and Dry-Flow Pure. The surface tension modifying agents are modified protein derivatives that reduce the surface tension of oil. Nonlimiting examples include Vegepol (sodium C8-16 isoalkylsuccinyl soy protein succinate) from Brooks Industries, NJ, and the like

c) saturated fatty acid contains less than 20 total carbon atoms e.g. stearic acid

The concentration of the 1,2-diol in the hair composition of the present disclosure may be from 0.1% to 12%, further may be from 0.2 to 5%; and further may be from 0.5 to 4%; and further may be from 1.0 to 3.0%. The concentration of the solid particle in the hair composition of the present disclosure may be from 0.1% to 10%, further may be from 0.5

to 5%, and further may be from 1.0 to 2.0%.

In an attempt, to identify the materials as sebum modifiers, which can change the physical characteristics of sebum such as their melting temperature, various mixtures of materials with sebum are prepared and measured using differential scanning calorimetry method. The following table provides the corresponding measurements.

Table 1: Example of Sebum Modifiers

| **Sebum Modifier Material** | **Endothermic Temperature Peak 1 (°C)** | **Endothermic Temperature Peak 2 (°C)** | **Endothermic Temperature Peak 3 (°C)** | **Endothermic Temperature Peak 4 (°C)** |
|---|---|---|---|---|
| Petrolatum | -23.00 | 3.00 | 16 | 40 |
| 1,2-dodecanediol | -18.00 | 4.90 | 21 | 36 |
| stearyl alcohol | -18.00 | 6.00 | 15 | 36 |
| cetyl alcohol | -18.00 | 4.00 | 18 | 26 |
| 1,2- decanediol | -20.00 | | | 42 |
| Stearic acid | -18.20 | 6.50 | | 39 |
| Zinc carbonate | 0.5 | 7 | | 75 |
| Zinc stearate | -4 | 8 | 21 | 97 |
| 1,2-octadecanediol | -4 | 10 | 20 | 51 |

In order to identify the appropriate solid particles, the absorption/adsorption characteristics of sebum on various solid particles are measured using contact angle method. The following table provides the corresponding measurements.

Table 2: Example of Solid Particles Physical Properties

| Material | Nonpolar | Polar | Total (Polar+ Nonpolar) | Work of Adhesion (sebum) | Spreading Coefficient (sebum) | Interfacial Tension (sebum) |
|---|---|---|---|---|---|---|
| Talc | 22.53 | 5.91 | 28.44 | 55.01 | 1.07 | 0.40 |
| Silica | 32.87 | 32.20 | 65.08 | 76.37 | 22.43 | 15.68 |
| Silica Silylate (hydrophobic silica) | 49.07 | 26.14 | 75.21 | 86.71 | 32.77 | 15.47 |
| Cellulose untreated | 27.50 | 41.00 | 68.50 | 74.24 | 20.30 | 21.23 |
| Zinc Carbonate | 44.83 | 26.38 | 71.21 | 83.78 | 29.84 | 14.40 |

PRODUCT FORMS

The hair care composition of the present disclosure may be a pre-wash composition, a rinse-off hair conditioner or a leave-on treatment. The hair care products comprise (a) 1,2-diol having a carbon chain with a length of more than 8 carbons and (b) solid particles which are able to absorb/adsorb sebum from scalp and/or hair. They also comprise an aqueous carrier.

1,2-DIOLS

The compositions of the pre-wash compositions (and the compositions of other types of hair care products) contain from 0.1 wt% to 12 wt% 1,2-diols having a carbon chain with length of more than 8 carbons. Non-limited examples include 1,2-dodecnediol, 1,2-decanediol, 1,2-octadecanediol.

Without being limited by theory, such 1,2-diols contribute to the modification of the melting characteristics of sebum making it more available to be absorbed /adsorbed by the solid which is present in the composition.

SOLID PARTICLES

The compositions of the pre-wash compositions (and the composition of other types of hair care products) contain from 0.1 wt% to 10 wt% solid particles, which are able to absorb/adsorb sebum from scalp and/or hair) wherein,

(1) the interfacial tension between the solid particle and sebum is from 5 to 18 dyn/cm;
(2) the sebum exhibits spreading coefficient on the solid, which is greater than 22 dyn/cm; and
(3) the work adhesion of the sebum to the solid particle, which is greater than 75 dyn/cm.

Non-limited examples of such solid particles include zinc carbonate, hydrophobically-modified silica, and mixtures thereof. Starches hydrophobically modified to have a high capacity for loading oils. Such starches can be modified with alkyl or alkenyl substituted dicarboxylic acids. Such materials may contain counter-ions, for example metals such as aluminum. A preferred such material is Natrasorb HFB available from National Starch and Chemical Company, U.S. A, which contains aluminum starch octenyl succinate. Other suitable materials from National Starch and Chemical include Natrasorb Bath, Dry-Flow PC, Dry-How XT, and Dry-Flow Pure. The surface tension modifying agents are modified protein derivatives that reduce the surface tension of oil. Nonlimiting examples include Vegepol (sodium C8-16 isoalkylsuccinyl soy protein succinate) from Brooks Industries, NJ, and the like.

## AQUEOUS CARRIER

The hair care composition comprises an aqueous carrier. Accordingly, the formulations of the hair care composition can be in the form of pourable liquids (under ambient conditions). Such compositions will therefore typically comprise an aqueous carrier, which is present at a level of from 20 wt.% to 95 wt.%, or from 60 wt.% to 85 wt.%. The aqueous carrier may comprise water, or a miscible mixture of water and organic solvent, and in one aspect may comprise water with minimal or no significant concentrations of organic solvent, except as otherwise incidentally incorporated into the composition as minor ingredients of other components.

The aqueous carriers useful in the hair care composition include water and water solutions of lower alkyl alcohols and polyhydric alcohols. The lower alkyl alcohols useful herein are monohydric alcohols having 1 to 6 carbons, in one aspect, ethanol and isopropanol. The polyhydric alcohols useful herein include propylene glycol, dipropylene glycol, hexylene glycol, glycerin, and propane diol.

A. PRE-WASH COMPOSITIONS

A pre-wash composition comprises of (a) 1,2-diol having a carbon chain with a length of more than 8 carbons, (b) solid particles which are able to absorb/adsorb sebum from scalp and/or hair and (c) An aqueous carrier. In addition, pre-wash composition may comprise other optional ingredients such as silicone or organic conditioning agents, hair health actives, anti-dandruff actives, and other ingredients.

The conditioning agent in liquid form should be added in moderate quantities, for example, less than 3%, in order to preserve the absorbing ability of the solid particles of the composition.

The pre-wash treatment of scalp and hair, using a pre-wash composition, is applied to hair and optionally rinsed. Then, washing with shampoo is performed at subsequent time (within a minute after the application of the pre-wash composition or even hours or days after the application of the pre-wash composition). A clarifying shampoo is a hair cleansing composition that is substantially free of insoluble conditioning agent as compared to conditioning shampoo that may contain such agents.

The pre-wash involves the application of a 1% w/w solution of the materials in a mixture of water, emulsifier and a thickener (Sepigel 305). Such materials may include 1,2-decanediol, 1,2-dodecanediol, 1,2-octanediol for 1-2-diols and silica silylate, salicylic acid, 2,4-dihydroxy benzoic acid, 4-chlororesorcinol, 1,2,4-Trihydroxybenzene and zinc carbonate for solid particles.

### Emulsifiers

In cases where the hair care composition does not include a gel matrix, the 1,2-diol can be pre-emulsified before it is added in the hair care composition. Emulsifiers selection for each conditioning active is guided by the Hydrophilic-Lipophilic-Balance value (HLB value) of emulsifiers. Suitable range of HLB value is 6-16, more preferably 8-14. Emulsifiers with an HLB higher than 10 are water soluble. Emulsifiers with low HLB are lipid soluble. To obtain suitable HLB value, a mixture of two or more emulsifiers may be used. Suitable emulsifiers include non-ionic, cationic, anionic and amphoteric emulsifiers.

## RHEOLOGY MODIFIER/THICKENER

The hair care compositions mentioned above may also contain one or more rheology modifier/thickener to adjust the rheological characteristics of the composition for better feel, in-use properties and the suspending stability of the composition. For example, the rheological properties are adjusted so that the composition remains uniform during its storage and transportation and it does not drip undesirably onto other areas of the body, clothing or home furnishings during its use. Any suitable rheology modifier can be used. Further, the leave-on treatment may comprise from 0.01% to 3% of a rheology modifier, alternatively from 0.1% to 1% of a rheology modifier,

The one or more rheology modifier may be selected from the group consisting of polyacrylamide thickeners, cationically modified polysaccharides, associative thickeners, and mixtures thereof. Associative thickeners include a variety of material classes such as, for example: hydrophobically modified cellulose derivatives; hydrophobically modified alkoxylated urethane polymers, such as PEG-150/decyl alcohol/SMDI copolymer, PEG-150/stearyl alcohol/SMDI copolymer, polyurethane-39; hydrophobically modified, alkali swellable emulsions, such as hydrophobically modified polypolyacrylates, hydrophobically modified polyacrylic acids, and hydrophobically modified polyacrylamides; hydrophobically modified polyethers. These materials may have a hydrophobe that can be selected from cetyl, stearyl, oleayl, and combinations thereof, and a hydrophilic portion of repeating ethylene oxide groups with repeat units from 10-300, alternatively from 30-200, and alternatively from 40-150. Examples of this class include PEG-120-methylglucose dioleate, PEG-(40 or 60) sorbitan tetraoleate, PEG-150 pentaerythrityl tetrastearate, PEG-55 propylene glycol oleate, PEG-150 distearate.

Non-limiting examples of additional rheology modifiers include acrylamide/ammonium acrylate copolymer (and)polyisobutene (and) polysorbate 20; acrylamide/sodium acryloyldimethyl taurate copolymer/ isohexadecane/ polysorbate 80; acrylates copolymer; acrylates/beheneth-25 methacrylate copolymer; acrylates/C10-C30 alkyl acrylate crosspolymer; acrylates/steareth-20 itaconate copolymer; ammonium polyacrylate/Isohexadecane/PEG-40 castor oil; C12-16 alkyl PEG-2 hydroxypropylhydroxyethyl ethylcellulose (HM-EHEC); carbomer; crosslinked polyvinylpyrrolidone (PVP); dibenzylidene sorbitol; hydroxyethyl ethylcellulose (EHEC); hydroxypropyl methylcellulose (HPMC); hydroxypropyl methylcellulose (HPMC); hydroxypropylcellulose (HPC); methylcellulose (MC); methylhydroxyethyl cellulose (MEHEC); PEG-150/decyl alcohol/SMDI copolymer; PEG-150/stearyl alcohol/SMDI copolymer; polyacrylamide/C13-14 isoparaffin/laureth-7; polyacrylate 13/polyisobutene/polysorbate 20; polyacrylate crosspolymer-6; polyamide-3; polyquaternium-37 (and) hydrogenated polydecene (and) trideceth-6; polyurethane-39; sodium acrylate/acryloyldimethyltaurate/dimethylacrylamide; crosspolymer (and) isohexadecane (and) polysorbate 60; sodium polyacrylate. Exemplary commercially-available rheology modifiers include ACULYN™ 28, Klucel M CS, Klucel H CS, Klucel G CS, SYLVACLEAR AF1900V, SYLVACLEAR PA1200V, Benecel E10M, Benecel K35M, Optasense RMC70, ACULYN™33, ACULYN™46, ACULYN™22, ACULYN™44, Carbopol Ultrez 20, Carbopol Ultrez 21, Carbopol Ultrez 10, Carbopol 1342, Sepigel™ 305, Simulgel™600, Sepimax Zen, and/or combinations thereof.

A non exclusive list of suitable thickeners for use herein include xanthan, guar, hydroxypropyl guar, scleroglucan, methyl cellulose, ethyl cellulose (commercially available as Aquacote (Registered trademark), hydroxyethyl cellulose (Natrosol (Registered trademark), carboxymethyl cellulose, hydroxypropylmethyl cellulose, microcrystalline cellulose, hydroxybutylmethyl cellulose, hydroxypropyl cellulose (Klucel (Registered trademark), hydroxyethyl ethyl cellulose, cetyl hydroxyethyl cellulose (Natrosol (Registered trademark Plus 330), N-vinylpyrollidone (Povidone (Registered trademark), Acrylates / Ceteth-20 Itaconate Copolymer (Structure (Registered trademark 3001), hydroxypropyl starch phosphate (Structure (Registered trademark ZEA), polyethoxylated urethanes or polycarbamyl polyglycol ester (e.g. PEG-150/Decyl/SMDI copolymer = Aculyn (Registered trademark 44, PEG-150/Stearyl/SMDI copolymer = Aculyn 46 (Registered trademark ), trihydroxystearin (Thixcin (Registered trademark) acrylates copolymer (e.g. Aculyn (Registered trademark 33) or hydrophobically modified acrylate copolymers (e.g. Acrylates / Steareth-20 Methacrylate Copolymer = Aculyn (Registered trademark 22), and fatty alcohols, such as cetyl and stearyl alcohol, and combinations thereof.

## CONDITIONER COMPOSITION

The hair care composition of the present disclosure can be a hair conditioner. The hair conditioner composition delivers consumer desired benefits such as wet feel, combability, color retention, protection against hair damage, damage repair, dry feel, anti-frizz benefits, etc. shampooing in addition to scalp anti-dandruff efficacy benefit.

The conditioner composition comprises (a) 1,2-diol having a carbon chain with a length of more than 8 carbons (b) solid particles which are able to absorb/adsorb sebum from scalp and/or hair; and an aqueous carrier. In addition, it may comprise other optional ingredients such as silicone or organic conditioning agents, hair health actives, anti-dandruff actives, and other ingredients.

Hair conditioners are typically applied on hair after rinsing the shampoo composition from the hair. The conditioner composition described herein delivers consumer desired hair conditioning in addition to anti-dandruff benefits.

The conditioner composition described herein may also comprise a conditioner gel matrix comprising (1) one

or more high melting point fatty compounds, (2) a cationic surfactant system, and (3) a second aqueous carrier. After applying to the hair a conditioner composition, the conditioner is rinsed from the hair using water.

**A.** CATIONIC SURFACTANT SYSTEM

The conditioner gel matrix of the conditioner composition includes a cationic surfactant system. The cationic surfactant system can be one cationic surfactant or a mixture of two or more cationic surfactants. The cationic surfactant system can be selected from: mono-long alkyl quaternized ammonium salt; a combination of mono-long alkyl quaternized ammonium salt and di-long alkyl quaternized ammonium salt; mono-long alkyl amidoamine salt; a combination of mono-long alkyl amidoamine salt and di-long alkyl quaternized ammonium salt, a combination of mono-long alkyl amindoamine salt and mono-long alkyl quaternized ammonium salt.
The cationic surfactant system can be included in the composition at a level by weight of from 0.1% to 10%, from 0.5% to 8%, from 0.8 % to 5%, and from 1.0% to 4%.

Mono-long alkyl quaternized ammonium salt

The monoalkyl quaternized ammonium salt cationic surfactants useful herein are those having one long alkyl chain which has 22 carbon atoms and in may be a C22 alkyl group. The remaining groups attached to nitrogen are independently selected from an alkyl group of from 1 to 4 carbon atoms or an alkoxy, polyoxyalkylene, alkylamido, hydroxyalkyl, aryl or alkylaryl group having up to 4 carbon atoms.
Mono-long alkyl quaternized ammonium salts useful herein are those having the formula (I):

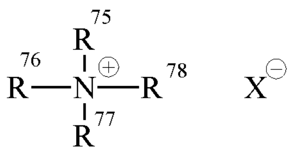

(I)

wherein one of $R^{75}$, $R^{76}$, $R^{77}$ and $R^{78}$ is selected from an alkyl group of 22 carbon atoms or an aromatic, alkoxy, polyoxyalkylene, alkylamido, hydroxyalkyl, aryl or alkylaryl group having up to 30 carbon atoms; the remainder of $R^{75}$, $R^{76}$, $R^{77}$ and $R^{78}$ are independently selected from an alkyl group of from 1 to 4 carbon atoms or an alkoxy, polyoxyalkylene, alkylamido, hydroxyalkyl, aryl or alkylaryl group having up to 4 carbon atoms; and $X^-$ is a salt-forming anion such as those selected from halogen, (e.g. chloride, bromide), acetate, citrate, lactate, glycolate, phosphate, nitrate, sulfonate, sulfate, alkylsulfate, and alkyl sulfonate radicals. The alkyl groups can contain, in addition to carbon and hydrogen atoms, ether and/or ester linkages, and other groups such as amino groups. The longer chain alkyl groups, e.g., those of 22 carbons, or higher, can be saturated or unsaturated. One of $R^{75}$, $R^{76}$, $R^{77}$ and $R^{78}$ can be selected from an alkyl group of 22 carbon atoms, the remainder of $R^{75}$, $R^{76}$, $R^{77}$ and $R^{78}$ are independently selected from $CH_3$, $C_2H_5$, $C_2H_4OH$, and mixtures thereof; and X is selected from the group consisting of Cl, Br, $CH_3OSO_3$, $C_2H_5OSO_3$, and mixtures thereof.
Nonlimiting examples of such mono-long alkyl quaternized ammonium salt cationic surfactants include: behenyl trimethyl ammonium salt.

**Mono-long alkyl amidoamine salt**

Mono-long alkyl amines are also suitable as cationic surfactants. Primary, secondary, and tertiary fatty amines are useful. Particularly useful are tertiary amido amines having an alkyl group of 22 carbons. Exemplary tertiary amido amines include: behenamidopropyldimethylamine, behenamidopropyldiethylamine, behenamidoethyldiethylamine, behenamidoethyldimethylamin. Useful amines in the present disclosure are disclosed in U.S. Patent 4,275,055, Nachtigal, et al. These amines can also be used in combination with acids such as ℓ-glutamic acid, lactic acid, hydrochloric acid, malic acid, succinic acid, acetic acid, fumaric acid, tartaric acid, citric acid, ℓ-glutamic hydrochloride, maleic acid, and mixtures thereof; and may be ℓ-glutamic acid, lactic acid, and/or citric acid. The amines herein can be partially neutralized with any of the acids at a molar ratio of the amine to the acid of from 1 : 0.3 to 1 : 2, and/or from 1 : 0.4 to 1 : 1.

Di-long alkyl quaternized ammonium salt

Di-long alkyl quaternized ammonium salt can be combined with a mono-long alkyl quaternized ammonium salt or mono-long alkyl amidoamine salt. It is believed that such combination can provide easy-to rinse feel, compared to single use of a monoalkyl quaternized ammonium salt or mono-long alkyl amidoamine salt. In such combination with a

mono-long alkyl quaternized ammonium salt or mono-long alkyl amidoamine salt, the di-long alkyl quaternized ammonium salts are used at a level such that the wt% of the dialkyl quaternized ammonium salt in the cationic surfactant system is in the range of from 10% to 50%, and/or from 30% to 45%.

The di-long alkyl quaternized ammonium salt cationic surfactants useful herein are those having two long alkyl chains having 22 carbon atoms. The remaining groups attached to nitrogen are independently selected from an alkyl group of from 1 to 4 carbon atoms or an alkoxy, polyoxyalkylene, alkylamido, hydroxyalkyl, aryl or alkylaryl group having up to 4 carbon atoms.

Di-long alkyl quaternized ammonium salts useful herein are those having the formula (II):

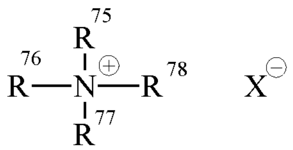

(II)

wherein two of $R^{75}$, $R^{76}$, $R^{77}$ and $R^{78}$ is selected from an alkyl group of from 22 carbon atoms or an aromatic, alkoxy, polyoxyalkylene, alkylamido, hydroxyalkyl, aryl or alkylaryl group having up to 30 carbon atoms; the remainder of $R^{75}$, $R^{76}$, $R^{77}$ and $R^{78}$ are independently selected from an alkyl group of from 1 to 4 carbon atoms or an alkoxy, polyoxyalkylene, alkylamido, hydroxyalkyl, aryl or alkylaryl group having up to 4 carbon atoms; and $X^-$ is a salt-forming anion such as those selected from halogen, (e.g. chloride, bromide), acetate, citrate, lactate, glycolate, phosphate, nitrate, sulfonate, sulfate, alkylsulfate, and alkyl sulfonate radicals. The alkyl groups can contain, in addition to carbon and hydrogen atoms, ether and/or ester linkages, and other groups such as amino groups. The longer chain alkyl groups, e.g., those of 22 carbons, or higher, can be saturated or unsaturated. One of $R^{75}$, $R^{76}$, $R^{77}$ and $R^{78}$ can be selected from an alkyl group of from 22 carbon atoms, the remainder of $R^{75}$, $R^{76}$, $R^{77}$ and $R^{78}$ are independently selected from $CH_3$, $C_2H_5$, $C_2H_4OH$, and mixtures thereof; and X is selected from the group consisting of Cl, Br, $CH_3OSO_3$, $C_2H_5OSO_3$, and mixtures thereof.

Such dialkyl quaternized ammonium salt cationic surfactants include, for example, dialkyl (C22) dimethyl ammonium chloride, ditallow alkyl dimethyl ammonium chloride, dihydrogenated tallow alkyl dimethyl ammonium chloride. Such dialkyl quaternized ammonium salt cationic surfactants also include, for example, asymmetric dialkyl quaternized ammonium salt cationic surfactants.

## B. HIGH MELTING POINT FATTY COMPOUND

The conditioner gel matrix of the conditioner composition includes one or more high melting point fatty compounds. The high melting point fatty compounds useful herein may have a melting point of 25°C or higher, and is selected from the group consisting of fatty alcohols, fatty acids, fatty alcohol derivatives, fatty acid derivatives, and mixtures thereof. It is understood by the artisan that the compounds disclosed in this section of the specification can in some instances fall into more than one classification, e.g., some fatty alcohol derivatives can also be classified as fatty acid derivatives. However, a given classification is not intended to be a limitation on that particular compound, but is done so for convenience of classification and nomenclature. Further, it is understood by the artisan that, depending on the number and position of double bonds, and length and position of the branches, certain compounds having certain carbon atoms may have a melting point of less than 25°C. Such compounds of low melting point are not intended to be included in this section. Nonlimiting examples of the high melting point compounds are found in International Cosmetic Ingredient Dictionary, Fifth Edition, 1993, and CTFA Cosmetic Ingredient Handbook, Second Edition, 1992.

Among a variety of high melting point fatty compounds, fatty alcohols are suitable for use in the conditioner composition. The fatty alcohols useful herein are those having from 14 to 30 carbon atoms, from 16 to 22 carbon atoms. These fatty alcohols are saturated and can be straight or branched chain alcohols. Suitable fatty alcohols include, for example, cetyl alcohol, stearyl alcohol, behenyl alcohol, and mixtures thereof.

High melting point fatty compounds of a single compound of high purity can be used. Single compounds of pure fatty alcohols selected from the group of pure cetyl alcohol, stearyl alcohol, and behenyl alcohol can also be used. By "pure" herein, what is meant is that the compound has a purity of at least 90%, and/or at least 95%. These single compounds of high purity provide good rinsability from the hair when the consumer rinses off the composition.

The high melting point fatty compound can be included in the conditioner composition at a level of from 0.1% to 20%, alternatively from 1% to 15%, and alternatively from 1.5% to 8% by weight of the composition, in view of providing improved conditioning benefits such as slippery feel during the application to wet hair, softness and moisturized feel on dry hair.

## LEAVE-ON TREATMENT

The hair care composition of the present disclosure can be a leave-on treatment. The leave-on treatment composition delivers consumer desired hair conditioning or styling benefit in addition to scalp anti-dandruff efficacy benefit.

The leave-on treatment composition comprises (a) 1,2-diol having a carbon chain with a length of more than 8 carbons (b) solid particles which are able to absorb/adsorb sebum from scalp and/or hair; and an aqueous carrier. The leave-on treatment may also comprise (1) one or more rheology modifiers. In addition, it may comprise other optional ingredients such as silicone or organic conditioning agents, thickners, hair health actives, anti-dandruff actives, and other ingredients.

Accordingly, the formulations of the leave-on treatment can be in the form of pourable liquids (under ambient conditions).

In cases where the leave-on composition does not include a gel matrix, it is preferred that the 1,2-diol of the composition is pre-emulsified before added in the hair care composition. In cases where the leave-on composition does not include a gel matrix, it is preferred that the composition also comprises a rheology modifier/thickener.

In the present disclosure, the leave-on treatment may involve the application of a 1% w/w solution of the materials in a mixture of water, emulsifier and a thickener (Sepigel 305). Preferred materials include 1,2-decanediol, 1,2-dodecanediol, 1,2-octanediol for 1-2-diols and silica silylate, salicylic acid, 2,4-dihydroxy benzoic acid, 4-chlororesorcinol, 1,2,4-Trihydroxybenzene and zinc carbonate for solid particles.

## pH

The hair care compositions mentioned above may also comprise one or more pH adjusting material. The compositions may have a pH in the range from 2 to 10, at 25°C. The rinse-off conditioner composition, and/or leave-on treatment may have a pH in the range of from 2 to 6, alternatively from 3.5 to 5, alternatively from 5.25 to 7.

The hair care compositions mentioned above may further comprise one or more pH buffering agent. Suitable buffering agents are well known in the art and include for example ammonia/ammonium acetate mixture and monoethanolamine (MEA). The rinse-off conditioner composition may comprise citric acid, wherein the citric acid acts as a buffer.

## OPTIONAL INGREDIENTS

The conditioner compositions, pre-wash compositions and/or leave-on treatments described herein may optionally comprise one or more additional components known for use in hair care or personal care products, provided that the additional components are physically and chemically compatible with the essential components described herein, or do not otherwise unduly impair product stability, aesthetics or performance. Such additional components are most typically those described in reference books such as the CTFA Cosmetic Ingredient Handbook, Second Edition, The Cosmetic, Toiletries, and Fragrance Association, Inc. 1988, 1992. Individual concentrations of such additional components may range from 0.001 wt.% to 10 wt.% by weight of the hair care compositions.

Non-limiting examples of additional components for use in the hair care compositions include conditioning agents, natural cationic deposition polymers, synthetic cationic deposition polymers, other anti-dandruff agents, particles, suspending agents, paraffinic hydrocarbons, propellants, viscosity modifiers, dyes, non-volatile solvents or diluents (water-soluble and water-insoluble), pearlescent aids, foam boosters, additional surfactants or nonionic cosurfactants, pediculocides, pH adjusting agents, perfumes, preservatives, proteins, skin active agents, sunscreens, UV absorbers, and vitamins.

### 1. Conditioning Agent

The hair care compositions may comprise one or more conditioning agents. Conditioning agents include materials that are used to give a particular conditioning benefit to hair. The conditioning agents useful in the hair care compositions of the present disclosure typically comprise a water-insoluble, water-dispersible, non-volatile, liquid that forms emulsified, liquid particles. Suitable conditioning agents for use in the hair care composition are those conditioning agents characterized generally as silicones , organic conditioning oils or combinations thereof, or those conditioning agents which otherwise form liquid, dispersed particles in the aqueous surfactant matrix.

One or more conditioning agents are present from 0.01 wt.% to 10 wt.%, from 0.1 wt.% to 8 wt.%, and from 0.2 wt.% to 4 wt.%, by weight of the composition.

### Silicone Conditioning Agent

The compositions of the present disclosure may contain one or more silicone conditioning agents. Examples

of the silicones include dimethicones, dimethiconols, cyclic silicones, methylphenyl polysiloxane, and modified silicones with various functional groups such as amino groups, quaternary ammonium salt groups, aliphatic groups, alcohol groups, carboxylic acid groups, ether groups, epoxy groups, sugar or polysaccharide groups, fluorine-modified alkyl groups, alkoxy groups, or combinations of such groups. Such silicones may be soluble or insoluble in the aqueous (or non-aqueous) product carrier. In the case of insoluble liquid silicones, the polymer can be in an emulsified form with droplet size of 10 nm to 30 micrometers

**Organic Conditioning Materials**

The conditioning agent of the compositions of the present disclosure may also comprise at least one organic conditioning material such as oil or wax, either alone or in combination with other conditioning agents, such as the silicones described above. The organic material can be nonpolymeric, oligomeric or polymeric. It may be in the form of oil or wax and may be added in the formulation neat or in a pre-emulsified form. Some non-limiting examples of organic conditioning materials include, but are not limited to: i) hydrocarbon oils; ii) polyolefins, iii) fatty esters, iv) fluorinated conditioning compounds, v) fatty alcohols, vi) alkyl glucosides and alkyl glucoside derivatives; vii) quaternary ammonium compounds; viii) polyethylene glycols and polypropylene glycols having a molecular weight of up to 2,000,000 including those with CTFA names PEG-20 200, PEG-400, PEG-600, PEG-1000, PEG-2M, PEG-7M, PEG-14M, PEG-45M and mixtures thereof.

**Benefit Agents**

The hair care composition may further comprise one or more additional benefit agents. The benefit agents comprise a material selected from the group consisting of anti-dandruff agents, antifungal agents, anti-itch agents, anti-bacterial agents, anti-microbial agents, moisturization agents, anti-oxidants, vitamins, lipid soluble vitamins, perfumes, brighteners, enzymes, sensates, attractants, dyes, pigments, bleaches, and mixtures thereof.

The hair care compositions of the present disclosure may be presented in typical hair care formulations. They may be in the form of solutions, dispersion, emulsions, powders, talcs, encapsulated, spheres, spongers, solid dosage forms, foams, and other delivery mechanisms. The compositions of the present disclosure may be hair tonics, leave-on hair products such as treatment, and styling products, rinse-off hair products such as hair conditioners, and treatment products; and any other form that may be applied to hair.

The hair care compositions may be provided in the form of a porous, dissolvable solid structure, such as those disclosed in U.S. Patent Application Publication Nos. 2009/0232873; and 2010/0179083. Accordingly, the hair care compositions comprise a chelant, a buffer system comprising an organic acid, from 23% to 75% surfactant; from 10% to 50% water soluble polymer; and optionally, from 1% to 15% plasticizer; such that the hair care composition is in the form of a flexible porous dissolvable solid structure, wherein said structure has a Percent open cell content of from 80% to 100%.

The hair care compositions may be in the form of a porous dissolvable solid structure comprising a chelant; a buffer system comprising an organic acid from 23% to 75% surfactant; wherein said surfactant has an average ethoxylate/alkyl ratio of from 0.001 to 0.45; from 10% to 50% water soluble polymer; and from 1% to 15% plasticizer; and wherein said article has a density of from 0.03 g/cm$^3$ to 0.20 g/cm$^3$.

The hair care compositions may be in the form of a viscous liquid comprising a chelant; a buffer system comprising an organic acid from 5-20% surfactant and a polycarboxylate rheology modifier; wherein the polycarboxylate is specifically chosen to be effective at the high electrolyte levels resulting from the incorporation of the key buffer system and chelant used for this invention. Non-limiting examples include acrylates/C10-C30 alkyl acrylate crosspolymers such as Carbopol EDT2020, 1342,1382, etc. from Lubrizol. Rheology benefits of these actives may include stability, ease of dispensing, smoothness of spreading, etc.

The hair care compositions are generally prepared by conventional methods such as are known in the art of making the compositions. Such methods typically involve mixing of the ingredients in one or more steps to a relatively uniform state, with or without heating, cooling, application of vacuum, and the like. The compositions are prepared such as to optimize stability (physical stability, chemical stability, photostability) and/or delivery of the active materials. The hair care composition may be in a single phase or a single product, or the hair care composition may be in a separate phases or separate products. If two products are used, the products may be used together, at the same time or sequentially. Sequential use may occur in a short period of time, such as immediately after the use of one product, or it may occur over a period of hours or days.

EVALUATION METHODS

A. Sample Preparation for Differential Scanning Calorimetry (DSC) Measurement

A quantity of 1g of artificial sebum is mixed with 1g of 1.2-diol sebum modifier material at room temperature and heated at 40°C water bath for 2 minutes to a solution or a uniform mixture and the solution or uniform mixture is cooled at room temperature. The sample thus prepared is measured using differential scanning calorimetry method as described below.

B. Differential Scanning Calorimetry (DSC) Measurement

A DSC 204 Netzsch TASC 414/3A is used for the evaluation of 1,2-diol sebum modifiers. Each measurement is performed in triplicate. The samples consists of approximately 5.5 mg of 1:1 sebum: sebum modifier, as prepared by the method described in A above. The sample is placed into T-Zero aluminum DSC pans, and then covered with stainless steel meshes. A sand baseline and burn off is performed before running samples, and after every 10 samples. Each evaluation is conducted by varying the temperature of the sample between the values of -50°C to 300°C at a heating rate of 5°C/minute under 200 ml/min nitrogen purge. An empty pan of the same type is employed as a reference and tested under the same experimental conditions. The standard deviation of this method is less than 5%. Temperature measurements are taken at the endotherm peaks of physical changes in sebum components and curve-fitting energy integration is performed for the entire endotherm curve.

C. Measurement of solid particles physical properties using Contact Angle Method

A series of physical parameters of solid surfaces are important for selecting the materials appropriate materials for sebum modification.

a. Dispersion component of the surface tension. This is the component of solid surface tension that is related to intermolecular attraction caused by nonpolar dispersion forces.
b. Polar component of the surface tension. This is the component of solid surface tension that is related to polar forces, such as hydrogen bonding and ion-dipole forces.
c. Solid surface tension. This is the surface tension of a solid surface. This parameter cannot be measured directly, but must be determined by extrapolation of polymer melt or solution data to 100 % solids or by contact angles with liquids of known surface tension.
d. Surface energy. This is the excess free energy of surface molecules compared to those of the bulk material. It arises from unbalanced molecular cohesive forces at a surface that cause the surface to contract and behave like a film or membrane. The surface energy is expressed in energy/unit area, such as joules/$cm^2$.
e. Surface tension. This expresses the force necessary to break the surface of a film of a given length (units are force/length, such as dyn/cm or newtons/m); the same numerically as surface energy, but different units.

The measurement of the contact angle on solid particles can be used to determine the above-mentioned physical properties. Contact angle is the interior angle that a drop makes between the substrate and a tangent drawn at the intersection between the drop and the substrate as shown in FIG. 1. This is the angle formed by a liquid at the three-phase boundary where a liquid, gas (air) and solid intersect.

Contact angles are determined using a ASTM D7490-13 (Standard Test Method for Measurement of the Surface Tension of Solid Coatings, Substrates and Pigments using Contact Angle Measurements).

The equipment used includes a goniometer consisting of a controlled light source, a stage to hold the tile, and a microscope or camera for viewing of the drop on the tile is required (First Ten Angstrom, Model 200, or equivalent). A 1-mL hypodermic syringe is also used equipped with a No. 27 blunt tipped stainless-steel needle, capable of providing 100 to 200 drops from 1mL. For this evaluation, the following reagents are used: (a) water - Type II reagent water (distilled) in accordance with ASTM Specification D1193-99, and (b) diiodomethane (99+% purity).

The method involves the following steps:

1. Formation of a pellet of solid particles on a ceramic tile. The ceramic tile should not be touched with the fingers or contaminated in any other way during position on goniometer stage.
2. Measurement of the contact angle on the solid particles in a constant temperature (73 ± 2°F) and humidity environment (50 ± 5%) using the goniometer. Contact angles are measured for each discrete droplet of water and diiodomethane on the tile as described in ASTM D7334 (or the manufacturer's literature for the specific instrument used). More specifically,

(a) The tile is positioned so that a drop of the liquid can be deposited without visible distortion of the drop shape due to movement.

(b) The tip of the hypodermic needle is set at the distance from the surface recommended by the manufacturer of the instrument (3 mm (1/8 inches for the specific instrument) and a drop of the test liquid 5μL in size is deposited on the tile. The drop size should be controlled to ± 0.1 μL.

(c) The camera or video device is focused so that the image of the drop can be captured.

(d) Two contact angle measurements (one on each drop edge) are made for a water droplet on the tile using commercial software designed to extract contact angles from movies or images. For example, First Ten Angstrom software version 2.1, build 363, or equivalent. If the contact angles on two edges are different by more than 4°, the values are eliminated and the test is repeated. The measurement is repeated 5 more times on new droplets. The contact angle for the tile is the average of the six angles measured for each side.

The image acquisition speed should capture at least 10 images from the time the drop hits the surface to the time it cannot be resolved from the surface of the sample. For the measurements reported herein, a capture rate of 900 images per second is utilized. The software described above extracts the contact angles from the video feed. The volume is also calculated using the same software under the sessile volume. The contact angles are plotted with the sessile volume plots. Enough time is allowed for the drop to wet out to equilibrium. However, in highly absorptive systems the drop absorbs into the material before equilibrium is achieved. In these cases, in which the drop rapidly (< 0.2 seconds) absorbs into the substrate, video is progressed until 2% of the volume of the drop absorbed into the substrate. The contact angle is recorded at that time point. This might mean the first resolved image in extremely fast absorbing systems if the second image shows more than 2% volume loss.

(e) The measurement of step (d) is repeated for diiodomethane droplet (instead of water droplet). The measurement is performed on the finished side of the clean untreated ceramic tile.

3. Calculation of physical properties of solids The contact angle values for water and diiodomethane are substituted into two separate expressions of the Owens-Wendt-Kaelble equation (one for each liquid). This results in three equations and two unknowns, which are then solved for the dispersion and polar components of surface tension (refer below paragraph for the equations and calculations).

Calculation of Surface Energy

The Owens-Wendt-Kaelble equation:

$$\sigma_{\mathrm{lg}}^{T}\frac{(\cos\theta+1)}{2}=\left(\sigma_{\mathrm{lg}}^{D}\gamma_{sg}^{D}\right)^{1/2}+\left(\sigma_{\mathrm{lg}}^{P}\gamma_{sg}^{P}\right)^{1/2}$$

where:

θ = the average contact angle for the test liquid on the test specimen,

$\sigma_{\mathrm{lg}}^{T}$ = the total surface tension of the test liquid in dyn/cm

$\sigma^D$ *and* $\sigma^P$ = the dispersive and polar components of the liquid surface tension, respectively, also in dyn/cm.

$\sigma_{sg}$ = the total surface energy of the test substrate in dyn/cm

$\sigma^D$ *and* $\sigma^P$ = the dispersive and polar components of the test substrate, respectively, also in dyn/cm.

| | Surface Tension ($\sigma_{lg}$) (dyn/cm) | | |
|---|---|---|---|
| Solvent | Nonpolar | Polar | Total |
| Diiodomethane | 50.8 | 0 | 50.8 |
| Water | 21.8 | 51.0 | 72.8 |

The Owens-Wendt-Kaelble equation is simplified to the following equation when a dispersive (nonpolar) solvent such as diiodomethane is used:

$$\sigma_{\mathrm{lg}}^{T}\frac{(\cos\theta+1)}{2}=\left(\sigma_{\mathrm{lg}}^{D}\gamma_{sg}^{D}\right)^{1/2}$$

The dispersive (nonpolar) component of surface energy ($\sigma^D_{sg}$) is determined. Surface tension properties for diiodomethane are known and included in the table above. The contact angle is experimentally determined using the method delineated above.

Upon inserting the calculated dispersive component of surface energy ($\sigma^D_{sg}$) for the substrate into the Owens-Wendt-Kaelble equation delineated above and using the contact angles determined for water, the polar component of surface energy ($\sigma^P_{sg}$) of the substrate is determined because the surface tension properties for water are known and included in the table above. The dispersive component ($\sigma^D_{sg}$) of the substrate is determined with diiodomethane as explained above.

Calculation of Thermodynamic Parameters

Thermodynamic parameters is calculated by inserting surface energy components into the following equations of state:

**Spreading Coefficient:**

The spreading coefficient (S) is determined by de Gennes (de Gennes, P.-G., Reviews of Modern Physics (1985), 57, 827-863).

$$S = \gamma^T_{sg} - \sigma^T_{lg} - \sigma_{sl}$$

Where $\sigma_{si}$ is the interfacial tension

**Interfacial Tension:**

The Owens-Wendt equation of state is used to determine interfacial tension $\sigma_{si}$ (D.K. Owens and R.C. Wendt, Journal of Applied Polymer Science (1969), 13, 1741-1747).

$$\sigma^T_{sl} = \gamma^T_{sg} + \sigma^T_{lg} - 2\left(\sigma^D_{lg}\gamma^D_{sg}\right)^{1/2} - 2\left(\sigma^P_{lg}\gamma^P_{sg}\right)^{1/2}$$

**Work of Adhesion:**

The work of adhesion (W) using the Dupré equation of state (A. Dupré, Theorie Mechanique de la Chaleur; Gauthier-Villars: Paris, 1869; pp 36W).

$$W = \gamma^T_{sg} + \sigma^T_{lg} - \sigma_{sl}$$

D. METHOD OF DETERMINATION OF SEBUM REMOVAL

Method of Preparing Sebum-fluorescence Dye

An amount of 20g of artificial sebum is mixed with 0.03g of Tinopal B (Benzoxazole, 2,2'-(2,5-thiophenediyl) bis[5-(1,1-dimethylethyl)], from BASF). The mixture is prepared in a brown vial to prevent light exposure and heated to 54°C using water bath to melt and mix the components. Artificial Sebum Composition is prepared by adding materials mentioned in below table and then heated to 54 °C using water bath, to make it a uniform mixture.

| Raw Material | Weight % | Amount added (for 100g) |
|---|---|---|
| Stearic Acid | 14 | 14 |
| Oleic acid | 8 | 8 |
| Squalene | 12 | 12 |

(continued)

| Raw Material | Weight % | Amount added (for 100g) |
|---|---|---|
| Cetyl Palmitate | 12 | 12 |
| Isostearyl Isostearate | 12 | 12 |
| Trioctanoin | 20 | 20 |
| Caprylic Capric Triglyceride | 20 | 20 |
| Cholesterol | 2 | 2 |

Method of Treating Hair with Aqueous Pre-Wash Composition

An amount of 0.20 g of the sebum-fluorescence dye mixture is applied and massaged onto hair switch onto natural virgin brown hair switches weighing 4.0 g via a syringe (dosage 0.05g of sebum-fluorescence per g of hair). Immediately afterwards, an image of the hair switch is acquired using a digital single-lens reflex camera with parallel polarizers (image at to) under 256 nm UV light having power of 8 W. A quantity of 0.4 g of the aqueous pre-wash composition is then applied, spread on hair switch and is left on the hair switch under 25°C and 50% relative humidity for 30 minutes. Then, the hair is wetted with water and 0.4 g of shampoo is applied (dosage 0.1g of shampoo per g of hair). The shampoo is massaged into the hair for 10 seconds and rinsed with deionized water for 10 seconds at a flow rate of 20 ml/minute. The hair Switch is then allowed to air dry and images are taken using the same camera under the conditions described above (image at $t_w$). The hair switch in this case is also assessed by expert graders, as described below. The area of the image occupied by blue color intensity light due to sebum-fluorescence mixture is analyzed (selecting the entire hair switch) using 2D projection. For this analysis Java-based image processing program is used. Then, the mean projected area is determined for the hair switch at to ($A_{t0}$) and for the hair at $t_w$ ($A_{tw}$) and the sebum removal iscalculated using the equation given below. Each experiment is repeated with three hair switches and the results are averaged. The percent sebum removal is calculated using below equation:

$$\%\ \text{Sebum Removal} = 100 \text{ x } (A_{tw} / A_{t0})$$

The standard error of sebum removal is less than 10%.

E. EVALUATION OF CLEAN FEEL AND CLEAN APPEARANCE

Hair Switch Clean Appearance and Feel Assessment Method

The air dried treated hair switches are rated by ten expert graders in terms of clean (non-greasy) appearance and feel based on a 5-point scale, 5 being the best clean (no greasy) and 1 being the worst clean (very greasy).

EXAMPLES AND COMPOSITIONS

The following examples illustrate non-limiting examples of the invention described herein. The exemplified oxidative dyeing, rinse-off conditioner compositions can be prepared by conventional formulation and mixing techniques. All parts, percentages, and ratios herein are by weight unless otherwise specified. Some components may come from suppliers as dilute solutions. The amount stated reflects the weight percent of the active material, unless otherwise specified.

PRE-WASH COMPOSITIONS

Table 1: Examples of Aqueous Pre-Wash Treatment Compositions

| Examples | Pre-Wash treatment Control | Comparative Example I | Comparative Example II | Comparative Example III | Comparative Example IV | Comparative Example V | Comparative Example VI | Example VII |
|---|---|---|---|---|---|---|---|---|
| Raw Material | (wt. / wt.) % | (wt. / wt.) % | (wt. / wt.) % | (wt. / wt.) % | (wt. / wt.) % | (wt. / wt.) % | (wt. / wt.) % | (wt. / wt.) % |
| Distilled Water | QS | QS | QS | QS | QS | QS | QS | QS |
| Polyacrylamide & C13-14 Isoparaffin & Laureth-7 (Sepigel 305) | 0.75 | 0.75 | 0.75 | 0.75 | 0.75 | 0.75 | 0.75 | 0.75 |
| Perfume | 0.2 | 0.2 | 0.2 | 0.2 | 0.2 | 0.2 | 0.2 | 0.2 |
| 1,2-decanediol | 0 | 1.0 | 0 | 0 | 0 | 0 | 1.0 | 1.0 |
| Cellulose | 0 | 0 | 1.0 | 0 | 0 | 0 | 1.0 | 0 |
| Zinc carbonate | 0 | 0 | 0 | 1.6 | 0 | 0 | 0 | 1.6 |
| Hydrophobic silica | 0 | 0 | 0 | 0 | 0.5 | 0 | 0 | 0 |
| 1,2-dodecanediol | 0 | 0 | 0 | 0 | 0 | 1.0 | 0 | 0 |
| Laureth-7 | 0.2 | 0.2 | 0.2 | 0.2 | 0.2 | 0.2 | 0.2 | 0.2 |
| PEG-100 Stearate | 0.2 | 0.2 | 0.2 | 0.2 | 0.2 | 0.2 | 0.2 | 0.2 |
| Cetearyl Glucoside & Cetearyl Alcohol | 0.2 | 0.2 | 0.2 | 0.2 | 0.2 | 0.2 | 0.2 | 0.2 |
| Methyl Paraben | 0.2 | 0.2 | 0.2 | 0.2 | 0.2 | 0.2 | 0.2 | 0.2 |
| Propyl Paraben | 0.15 | 0.15 | 0.15 | 0.15 | 0.15 | 0.15 | 0.15 | 0.15 |
| Benzyl Alcohol | 0.40 | 0.40 | 0.40 | 0.40 | 0.40 | 0.40 | 0.40 | 0.40 |
| Phenoxy Ethanol | 0.40 | 0.40 | 0.40 | 0.40 | 0.40 | 0.40 | 0.40 | 0.40 |
| % Sebum Removal at dose of 0.10g of composition for 1.0g of hair | 78 | 90 | 72 | 81 | 86 | 85 | 76 | 97 |
| Clean Feel Rating at dose of 0.10g of composition for 1.0g of hair (on 5 scale point with 5 as highest clean feel and 1 as lowest) | 2.0 | 3.5 | 2.0 | 3.0 | 3.5 | 3.0 | 2.0 | 4.5 |

(continued)

| Examples | Pre-Wash treatment Control | Comparative Example I | Comparative Example II | Comparative Example III | Comparative Example IV | Comparative Example V | Comparative Example VI | Example VII |
|---|---|---|---|---|---|---|---|---|
| Clean Look Rating at dose of 0.10g of composition for 1.0g of hair (on 5 scale point with 5 as highest clean look and 1 as lowest) | 2.0 | 3.8 | 3.2 | 3.0 | 3.5 | 3.0 | 1.0 | 4.5 |

| Examples | VIII | IX | Comparative Example X | Comparative Example XI |
|---|---|---|---|---|
| Raw Material | (wt. / wt.) % | (wt. / wt.) % | (wt. / wt.) % | (wt. / wt.) % |
| Distilled Water | QS | QS | QS | QS |
| Polyacrylamide & C13-14 Isoparaffin & Laureth-7 (Sepigel 305) | 0.75 | 0.75 | 0.75 | 0.75 |
| Perfume | 0.2 | 0.2 | 0.2 | 0.2 |
| 1,2-decanediol | 1 | 0 | 0 | 1.0 |
| Zinc carbonate | 0 | 1.6 | 0 | 0 |
| Hydrophobic silica | 0.5 | 0 | 0 | 0 |
| 1,2-dodecanediol | 0 | 1.0 | 1.0 | |
| Talc | 0 | 0 | 1.0 | 1.0 |
| Laureth-7 | 0.2 | 0.2 | 0.2 | 0.2 |
| PEG-100 Stearate | 0.2 | 0.2 | 0.2 | 0.2 |
| Cetearyl Glucoside & Cetearyl Alcohol | 0.2 | 0.2 | 0.2 | 0.2 |
| Methyl Paraben | 0.2 | 0.2 | 0.2 | 0.2 |
| Propyl Paraben | 0.15 | 0.15 | 0.15 | 0.15 |
| Benzyl Alcohol | 0.40 | 0.40 | 0.40 | 0.40 |
| Phenoxy Ethanol | 0.40 | 0.40 | 0.40 | 0.40 |
| % Sebum Removal at dose of 0.10g of composition for 1.0g of hair | 93 | 90 | 68 | 76 |
| Clean Feel Rating at dose of 0.10g of composition for 1.0g of hair (on 5 scale point with 5 as highest clean feel and 1 as lowest) | 4.0 | 3.5 | 1.0 | 2.0 |
| Clean Look Rating at dose of 0.10g of composition for 1.0g of hair (on 5 scale point with 5 as highest clean look and 1 as lowest) | 3.8 | 3.8 | 3.2 | 2.0 |

**Results:** Formula I, IV, V, VII, VIII, IX showed increase in % sebum removal than control pre-wash treatment.

The feel assessment results indicate that combinations of

(a) 1,2-decanediol and zinc carbonate;
(b) 1,2-decanediol and hydrophobic silica;

provide, not only sebum removal (resulting in clean benefit), but also clean feel benefit. This is shown by the feel comparison of (a) Example VII versus Example I and III (b) Example VIII versus Example I and III.

METHODS OF MAKING THE COMPOSITIONS

In the case of 1,2-diols, the sebum modifier is pre-emulsified before it is added in the hair care composition.

Method of making pre-emulsion

Making the emulsion of comprising components below is to pre-emulsify the sebum modifier before their addition to the pre-wash composition. A non-limiting example of a method of making is provided below. All oil soluble components are mixed in a vessel. Heat may be applied to allow mixture to liquidify. All water-soluble components are mixed in a

separate vessel and heated to same temperature as the oil phase. The oil phase and aqueous phase are mixed under a high shear mixer (example, Turrax mixer by IKA).

Method of Making Aqueous Pre-Wash Compositions

The Sepigel 305 is then added to the pre-emulsion, if needed, and the solution is mixed using a high-speed-mixer for 2-5 minutes at 1800-2300 rpm until a uniform soluble composition is obtained.

RINSE-OFF CONDITIONER FORMULATIONS

| | Rinse-off Conditioner Ex 1 |
|---|---|
| Ingredients | Wt% |
| Amodimethicone 10000 cps | 0.50 |
| Citric acid anhydrous | 0.13 |
| DL-Panthenol 56% solution | 0.054 |
| Panthenyl Ethyl ether | 0.03 |
| Perfume | 0.50 |
| Hydroxypropyl guar (Jaguar HP-105) | 0.350 |
| Quaternium-18 | 0.750 |
| Steramidopropyldimethylamine | 1.00 |
| Gryceryl stearate | 0.25 |
| Cetearyl alcohol and Polysorbate 60 Emulsion [1] | 0.50 |
| Cetyl alcohol | 1.20 |
| Stearyl alcohol | 0.80 |
| Benzyl alcohol | 0.40 |
| Methylchloroisothiazolinone /methylisothiazolinone | 0.033 |
| 1.2 decanediol | 1 |
| Zinc carbonate | 1.6 |
| Water Purified | QS to 100 |
| [1]. Lipowax P from Lipo | |

The formulations of the present disclosure may be present in typical hair care compositions. They may be in the form of solutions, dispersion, emulsions, powders, talcs, encapsulated, spheres, spongers, solid dosage forms, foams, and other delivery mechanisms. The composition of the present disclosure may be hair tonics, leave-on hair products such as conditioners, treatment, and styling products, and any other form that may be applied to the hair.

In the examples, all concentrations are listed as weight percent, unless otherwise specified and may exclude minor materials such as diluents, filler, and so forth. The listed formulations, therefore, comprise the listed components and any minor materials associated with such components. As is apparent to one of ordinary skill in the art, the selection of these minors will vary depending on the physical and chemical characteristics of the particular ingredients selected to make the hair care composition.

## Claims

**1.** A hair care composition comprising:

a) from 0.1 wt.% to 12 wt.% of a 1,2-diol having a carbon chain with a length of more than 8 carbons;
b) from 0.1 wt.% to 10 wt.% of a solid particle, the solid particle is selected from the group consisting of silica silylate (hydrophobic silica), zinc carbonate and mixtures thereof, wherein the total amount of solid particle ranges from 1 wt.% to 10 wt.%, wherein

(1) the interfacial tension between the solid particle and sebum is from 5 to 18 dyn/cm as measured using the Contact Angle Method as disclosed herein;
(2) the sebum exhibits spreading coefficient on the solid, which is greater than 22 dyn/cm as measured using the Contact Angle Method as disclosed herein; and
(3) the work of adhesion of the sebum to the solid particle, which is greater than 75 dyn/cm as measured using the Contact Angle Method as disclosed herein.

c) an aqueous carrier.

**2.** A hair care composition according to claim 1, wherein the 1,2-diol having a carbon chain length of more than 8 carbons is from 0.2 to 5%.

**3.** A hair care composition according to any preceding claims wherein the 1,2-diol having a carbon chain length of more than 8 carbons is from 0.5 to 4%.

**4.** A hair care composition according to any preceding claims wherein the 1,2-diol having a carbon chain length of more than 8 carbons is from 1.0 to 3.0%.

**5.** A hair care composition according to any preceding claims wherein the solid particle is from 0.5 to 5%.

**6.** A hair care composition according to any preceding claims wherein the solid particle is from 1.0% to 2.0%.

**7.** A hair care composition according to any preceding claims further comprising from 0.1 wt.% to 5 wt.% emulsifier selected from the group consisting of anionic, non-ionic, cationic and amphoteric.

**8.** A hair care composition according to any preceding claims wherein the aqueous carrier is from 20 wt.% to 95 wt.%.

**9.** A hair care composition according to any preceding claims wherein the aqueous carrier is from 60 wt.% to 85 wt.%.

**10.** A hair care composition according to any preceding claims wherein the hair care composition is a rinse off conditioner.

**11.** A hair care composition according to any preceding claims wherein the hair care composition is a leave on treatment.

**12.** A hair care composition according to any preceding claims wherein the hair care composition is a pre-wash composition.

## Patentansprüche

**1.** Haarpflegezusammensetzung, umfassend:

a) von 0,1 Gew.-% bis 12 Gew.-% ein 1,2-Diol mit einer Kohlenstoffkette mit einer Länge von mehr als 8 Kohlenstoffen;
b) von 0,1 Gew.-% bis 10 Gew.-% ein Feststoffteilchen, wobei das Feststoffteilchen ausgewählt ist aus der Gruppe bestehend aus Silicasilylat (hydrophobes Silica), Zinkcarbonat und Mischungen davon, wobei die Gesamtmenge an Feststoffteilchen im Bereich von 1 Gew.-% bis 10 Gew.-% liegt, wobei

(1) die Grenzflächenspannung zwischen dem Feststoffteilchen und Sebum von 5 bis 18 dyn/cm beträgt, wie unter Verwendung des Kontaktwinkelverfahrens, wie hierin offenbart, gemessen;
(2) das Sebum einen Ausbreitungskoeffizienten auf dem Feststoff vorweist, der größer als 22 dyn/cm ist, wie unter Verwendung des Kontaktwinkelverfahrens, wie hierin offenbart, gemessen; und
(3) die Adhäsionsarbeit des Sebums an das Feststoffteilchen, die größer als 75 dyn/cm ist, wie unter Verwendung des Kontaktwinkelverfahrens, wie hierin offenbart, gemessen.

c) einen wässrigen Träger.

**2.** Haarpflegezusammensetzung nach Anspruch 1, wobei das 1,2-Diol mit einer Kohlenstoffkettenlänge von mehr als 8 Kohlenstoffen 0,2 bis 5 % beträgt.

**3.** Haarpflegezusammensetzung nach einem der vorstehenden Ansprüche, wobei das 1,2-Diol mit einer Kohlenstoffkettenlänge von mehr als 8 Kohlenstoffen 0,5 bis 4 % beträgt.

**4.** Haarpflegezusammensetzung nach einem der vorstehenden Ansprüche, wobei das 1,2-Diol mit einer Kohlenstoffkettenlänge von mehr als 8 Kohlenstoffen 1,0 bis 3,0 % beträgt.

**5.** Haarpflegezusammensetzung nach einem der vorstehenden Ansprüche, wobei das Feststoffteilchen 0,5 bis 5 % beträgt.

**6.** Haarpflegezusammensetzung nach einem der vorstehenden Ansprüche, wobei Feststoffteilchen von 1,0 % bis 2,0 % beträgt.

**7.** Haarpflegezusammensetzung nach einem der vorstehenden Ansprüche, ferner umfassend von 0,1 Gew.-% bis 5 Gew.-% Emulgator, ausgewählt aus der Gruppe bestehend aus anionischen, nichtionischen, kationischen und amphoteren.

**8.** Haarpflegezusammensetzung nach einem der vorstehenden Ansprüche, wobei der wässrige Träger von 20 Gew.-% bis 95 Gew.-% beträgt.

**9.** Haarpflegezusammensetzung nach einem der vorstehenden Ansprüche, wobei der wässrige Träger von 60 Gew.-% bis 85 Gew.-% beträgt.

**10.** Haarpflegezusammensetzung nach einem der vorstehenden Ansprüche, wobei die Haarpflegezusammensetzung ein ausspülbarer Conditioner ist.

**11.** Haarpflegezusammensetzung nach einem der vorstehenden Ansprüche, wobei die Haarpflegezusammensetzung eine zum Belassen auf dem Haar bestimmte Behandlung ist.

**12.** Haarpflegezusammensetzung nach einem der vorstehenden Ansprüche, wobei die Haarpflegezusammensetzung eine Vorwaschzusammensetzung ist.

## Revendications

**1.** Composition pour soins capillaires comprenant :

a) de 0,1 % en poids à 12 % en poids d'un 1,2-diol ayant une chaîne carbonée avec une longueur de plus de 8 carbones ;
b) de 0,1 % en poids à 10 % en poids d'une particule solide, la particule solide est choisie dans le groupe constitué de silylate de silice (silice hydrophobe), carbonate de zinc et mélanges de ceux-ci, dans laquelle la quantité totale de la particule solide va de 1 % en poids à 10 % en poids, dans laquelle

(1) la tension interfaciale entre la particule solide et du sébum va de 5 à 18 dyn/cm telle que mesurée en utilisant le procédé d'angle de contact tel que décrit ici ;
(2) le sébum présente un coefficient d'étalement sur le solide, qui est supérieur à 22 dyn/cm tel que mesuré en utilisant le procédé d'angle de contact tel que décrit ici ; et
(3) le travail d'adhésion du sébum à la particule solide, qui est supérieur à 75 dyn/cm tel que mesuré en utilisant le procédé d'angle de contact tel que décrit ici.

c) un véhicule aqueux.

**2.** Composition pour soins capillaires selon la revendication 1, dans laquelle le 1,2-diol ayant une longueur de chaîne carbonée de plus de 8 carbones représente de 0,2 à 5 %.

3. Composition pour soins capillaires selon de quelconques revendications précédentes, dans laquelle le 1,2-diol ayant une longueur de chaîne carbonée de plus de 8 carbones représente de 0,5 à 4 %.

4. Composition pour soins capillaires selon de quelconques revendications précédentes, dans laquelle le 1,2-diol ayant une longueur de chaîne carbonée de plus de 8 carbones représente de 1,0 à 3,0 %.

5. Composition pour soins capillaires selon de quelconques revendications précédentes dans laquelle la particule solide représente de 0,5 à 5 %.

6. Composition pour soins capillaires selon de quelconques revendications précédentes dans laquelle la particule solide représente de 1,0 % à 2,0 %.

7. Composition pour soins capillaires selon de quelconques revendications précédentes comprenant en outre de 0,1 % en poids à 5 % en poids d'un émulsifiant choisi dans le groupe constitué d'anionique, non ionique, cationique et amphotère.

8. Composition pour soins capillaires selon de quelconques revendications précédentes dans laquelle le véhicule aqueux représente de 20 % en poids à 95 % en poids.

9. Composition pour soins capillaires selon de quelconques revendications précédentes dans laquelle le véhicule aqueux représente de 60 % en poids à 85 % en poids.

10. Composition pour soins capillaires selon de quelconques revendications précédentes dans laquelle la composition pour soins capillaires est un conditionneur à rincer.

11. Composition pour soins capillaires selon de quelconques revendications précédentes dans laquelle la composition pour soins capillaires est un traitement à laisser.

12. Composition pour soins capillaires selon de quelconques revendications précédentes dans laquelle la composition pour soins capillaires est une composition de prélavage.

FIG. 1

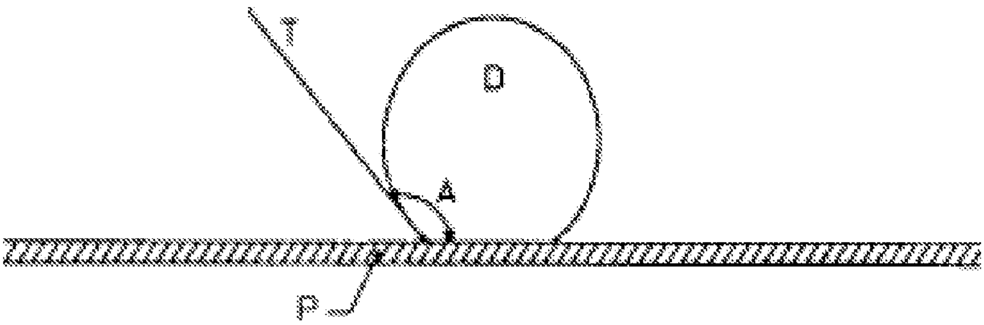


A – contact angle
D – drop of liquid
P – specimen
T – tangent at specimen surface

**FIG. 1 Measuring Angle of Contact**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011047421 A1
- US 2015118172 A1
- EP 3061501 A1
- WO 2017027603 A1
- US 4275055 A, Nachtigal
- US 20090232873
- US 20100179083


### Non-patent literature cited in the description


- International Cosmetic Ingredient Dictionary. 1993
- CTFA Cosmetic Ingredient Handbook. 1992
- **DE GENNES, P.-G.** *Reviews of Modern Physics,* 1985, vol. 57, 827-863
- **D.K. OWENS ; R.C. WENDT.** *Journal of Applied Polymer Science,* 1969, vol. 13, 1741-1747